(19) Europäisches Patentamt
European Patent Office
Office européen des brevets

(11) EP 3 298 007 B1

(12) EUROPEAN PATENT SPECIFICATION

(45) Date of publication and mention of the grant of the patent:
**22.07.2020 Bulletin 2020/30**

(21) Application number: **16730933.5**

(22) Date of filing: **18.05.2016**

(51) Int Cl.:
***C07D 409/10*** (2006.01) ***A61K 31/381*** (2006.01)
***A61P 3/10*** (2006.01)

(86) International application number:
**PCT/US2016/033071**

(87) International publication number:
**WO 2016/191173 (01.12.2016 Gazette 2016/48)**

(54) **ANHYDROUS CRYSTALLINE FORM OF (1S)-1,5-ANHYDRO-1-[3-[[5-(4-FLUOROPHENYL)-2-THIENYL]METHYL]-4-METHYLPHENYL]-D-GLUCITOL**

WASSERFREIE KRISTALLINE FORM VON (1S)-1,5-ANHYDRO-1-[3-[[5-(4-FLUORPHENYL) -2-THIENYL] METHYL]-4-METHYLPHENYL]-D-GLUCITOL

FORME CRISTALLINE ANHYDRE DE (1S)-1,5-ANHYDRO-1-[3-[[5-(4-FLUOROPHÉNYL)-2-THIÉNYL]MÉTHYL]-4-MÉTHYLPHÉNYL]-D-GLUCITOL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **22.05.2015 US 201562165265 P**

(43) Date of publication of application:
**28.03.2018 Bulletin 2018/13**

(73) Proprietor: **Janssen Pharmaceutica NV**
**2340 Beerse (BE)**

(72) Inventors:
- **BRESLIN, David T.**
  **Spring House, Pennsylvania 19477 (US)**
- **FAWZY, Nagy E.**
  **Spring House, Pennsylvania 19477 (US)**
- **SCHAEFER, John**
  **Spinghouse, Pennsylvania 19477 (US)**

(74) Representative: **McGuire, Gillian Margaret**
**Carpmaels & Ransford LLP**
**One Southampton Row**
**London WC1B 5HA (GB)**

(56) References cited:
**WO-A1-2008/069327** **WO-A1-2011/003976**
**WO-A1-2014/180872** **WO-A1-2015/158839**
**WO-A2-2010/043682** **WO-A2-2013/064909**
**CN-A- 104 230 907** **CN-A- 104 447 721**
**CN-A- 104 447 722** **CN-A- 104 693 192**
**CN-A- 105 001 214**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 298 007 B1

## Description

### CROSS REFERENCE TO RELATED APPLICATIONS

This application claims the benefit of U.S. Provisional Application 62/165,265, filed on May 22, 2015.

### FIELD OF THE INVENTION

The present disclosure is directed to an anhydrous crystalline form of (1S)-1,5-anhydro-1-[3-[[5-(4-fluorophenyl)-2-thienyl]methyl]-4-methylphenyl]-D-glucitol, pharmaceutical compositions containing said anhydrous crystalline form and its use in the treatment of glucose-related disorders such as Type 2 diabetes mellitus and Syndrome X. The invention is directed to processes as defined herein.

### BACKGROUND OF THE INVENTION

Diabetes mellitus is a medical term for the presence of elevated blood glucose. People with diabetes either don't produce insulin, produce too little insulin or do not respond to insulin, resulting in the build-up of glucose in the blood. The most common form of diabetes is Type 2 diabetes, once referred to as adult onset diabetes or non-insulin dependent diabetes (NIDDM), which may account for >90% of diabetes in adults. However, as the younger population becomes increasingly overweight or obese, Type 2 diabetes is becoming more prevalent in teens and children. Diabetes may also refer to gestational diabetes, Type 1 diabetes or autoimmune diabetes, once referred to as juvenile onset diabetes and type 1 1/2 diabetes, also referred to as latent-autoimmune diabetes in adults or LADA. Diabetes may occur because of poor dietary habits or lack of physical activity (e.g., sedentary lifestyle), genetic mutations, injury to the pancreas, drug (e.g., AIDS therapies) or chemical (e.g., steroid) exposure or disease (e.g., cystic fibrosis, Down syndrome, Cushing's syndrome). Two rare types of genetic defects leading to diabetes are termed maturity-onset diabetes of the young (MODY) and atypical diabetes mellitus (ADM).

Type 2 diabetes mellitus (non-insulin-dependent diabetes mellitus or NIDDM) is a metabolic disorder involving disregulation of glucose metabolism and insulin resistance, and long-term complications involving the eyes, kidneys, nerves, and blood vessels. Type 2 diabetes mellitus usually develops in adulthood (middle life or later) and is described as the body's inability to make either sufficient insulin (abnormal insulin secretion) or its inability to effectively use insulin (resistance to insulin action in target organs and tissues). More particularly, patients suffering from Type 2 diabetes mellitus have a relative insulin deficiency. That is, in these patients, plasma insulin levels are normal to high in absolute terms, although they are lower than predicted for the level of plasma glucose that is present.

Type 2 diabetes mellitus is characterized by the following clinical signs or symptoms: persistently elevated plasma glucose concentration or hyperglycemia; polyuria; polydipsia and / or polyphagia; chronic microvascular complications such as retinopathy, nephropathy and neuropathy; and macrovascular complications such as hyperlipidemia and hypertension which can lead to blindness, end-stage renal disease, limb amputation and myocardial infarction.

Syndrome X, also termed Insulin Resistance Syndrome (IRS), Metabolic Syndrome, or Metabolic Syndrome X, is a disorder that presents risk factors for the development of Type 2 diabetes mellitus and cardiovascular disease including glucose intolerance, hyperinsulinemia and insulin resistance, hypertriglyceridemia, hypertension and obesity.

The diagnosis of Type 2 diabetes mellitus includes assessment of symptoms and measurement of glucose in the urine and blood. Blood glucose level determination is necessary for an accurate diagnosis. More specifically, fasting blood glucose level determination is a standard approach used. However, the oral glucose tolerance test (OGTT) is considered to be more sensitive than fasted blood glucose level. Type 2 diabetes mellitus is associated with impaired oral glucose tolerance (OGT). The OGTT thus can aid in the diagnosis of Type 2 diabetes mellitus, although generally not necessary for the diagnosis of diabetes (EMANCIPATOR K, Am J Clin Pathol 1999 Nov; pp665-674, Vol. 112(5):665-74; Type 2 Diabetes Mellitus, Decision Resources Inc., March 2000). The OGTT allows for an estimation of pancreatic beta-cell secretory function and insulin sensitivity, which helps in the diagnosis of Type 2 diabetes mellitus and evaluation of the severity or progression of the disease (e.g., CAUMO, A., et al., J Clin Endocrinol Metab, 2000, pp 4396-4402, Vol. 85(11)). More particularly, the OGTT is extremely helpful in establishing the degree of hyperglycemia in patients with multiple borderline fasting blood glucose levels that have not been diagnosed as diabetics. In addition, the OGTT is useful in testing patients with symptoms of Type 2 diabetes mellitus where the possible diagnosis of abnormal carbohydrate metabolism has to be clearly established or refuted.

Thus, impaired glucose tolerance is diagnosed in individuals that have fasting blood glucose levels less than those required for a diagnosis of Type 2 diabetes mellitus, but have a plasma glucose response during the OGTT between normal and diabetics. Impaired glucose tolerance is considered a pre-diabetic condition, and impaired glucose tolerance (as defined by the OGTT) is a strong predictor for the development of Type 2 diabetes mellitus (HAFFNER, S.M., Diabet Med, 1997 Aug; 14 Suppl 3:S12-8).

Type 2 diabetes mellitus is a progressive disease associated with the reduction of pancreatic function and/or other insulin-related processes, aggravated by increased plasma glucose levels. Thus, Type 2 diabetes mellitus usually has a prolonged pre-diabetic phase and various pathophysiological mechanisms can lead to pathological hyperglycemia and impaired glucose tolerance, for instance, abnormalities in glucose utilization and effectiveness, insulin action and/or insulin production in the pre-diabetic state (GOLDBERG, R.B., Med Clin North Am , 1998 Jul; pp805-821, Vol. 82(4)).

The pre-diabetic state associated with glucose intolerance can also be associated with a predisposition to abdominal obesity, insulin resistance, hyperlipidemia, and high blood pressure, that is, Syndrome X (GROOP L, et al., Am J Hypertens, 1997 Sep;10(9 Pt 2):172S-180S; HAFFNER, S.M., J Diabetes Complications, 1997 Mar-Apr; pp69-76, Vol. 11(2); BECK-NIELSEN, H., et al., Diabet Med, 1996 Sep;13(9 Suppl 6):S78-84).

Thus, defective carbohydrate metabolism is pivotal to the pathogenesis of Type 2 diabetes mellitus and impaired glucose tolerance (DIUNNEEN, S.F., Diabet Med, 1997 Aug; 14 Suppl 3:S19-24). In fact, a continuum from impaired glucose tolerance and impaired fasting glucose to definitive Type 2 diabetes mellitus exists (RAMLO-HALSTED, B.A., et al., Prim Care, 1999 Dec; pp 771-789, Vol. 26(4)).

Early intervention in individuals at risk to develop Type 2 diabetes mellitus, focusing on reducing the pathological hyperglycemia or impaired glucose tolerance may prevent or delay the progression towards Type 2 diabetes mellitus and associated complications and/or Syndrome X. Therefore, by effectively treating impaired oral glucose tolerance and / or elevated blood glucose levels, one can prevent or inhibit the progression of the disorder to Type 2 diabetes mellitus or Syndrome X.

Typical treatment of glucose disorders including Type 2 diabetes mellitus and Syndrome X focuses on maintaining the blood glucose level as near to normal as possible and includes diet and exercise, and when necessary, treatment with anti-diabetic agents, insulin or a combination thereof. Type 2 diabetes mellitus that cannot be controlled by dietary management is treated with oral antidiabetic agents including, but not limited to, sulfonylureas (e.g., not limited to first generation: chlorpropamide, tolazamide, tolbutamide; second generation: glyburide, glipizide; and third generation: glimepiride), biguanides (e.g., metformin), thiazolidinediones (e.g., rosiglitazone, pioglitazone, troglitazone), alpha-glucosidase inhibitors (e.g., acarbose, miglitol), meglitinides (e.g., repaglinide), other insulin-sensitizing compounds, and /or other anti-obesity agents (e.g., orlistat or sibutramine). For Syndrome X, the anti-diabetic agents are additionally combined with pharmacological agents for the treatment of the concomitant co-morbidities (e.g., antihypertensives for hypertension, hypolipidemic agents for hyperlipidemia).

First-line therapies typically include metformin and sulfonylureas as well as thiazolidinediones. Metformin monotherapy is a first line choice, particularly for treating Type 2 diabetic patients who are also obese and / or dyslipidemic. Lack of an appropriate response to metformin is often followed by treatment with metformin in combination with sulfonylureas, thiazolidinediones, or insulin. Sulfonylurea monotherapy (including all generations of drugs) is also a common first line option. Another first line therapy choice may be thiazolidinediones. Patients who do not respond appropriately to oral anti-diabetic monotherapy, are given combinations of these agents. When glycemic control cannot be maintained with oral antidiabetics alone, insulin therapy is used either as a monotherapy, or in combination with oral antidiabetic agents. These same strategies, optionally in combination with additional strategies (e.g., anti-hypertensive) can be used for the treatment of Syndrome X.

In addition to antidiabetic agents, therapies may include add-on treatment with anti-obesity agents such as orlistat, a pancreatic lipase inhibitor, which prevents the breakdown and absorption of fat; or sibutramine, an appetite suppressant and inhibitor of the reuptake of serotonin, norepinephrine and dopamine in the brain. Other potential add-on anti-obesity agents include, but are not limited to, appetite-suppressants acting through adrenergic mechanisms such as benzphetamine, phenmetrazine, phentermine, diethylpropion, mazindol, sibutramine, phenylpropanolamine or, ephedrine; appetite-suppressant agents acting through serotonergic mechanisms such as quipazine, fluoxetine, sertraline, fenfluramine, or dexfenfluramine; appetite-suppressant agents acting through dopamine mechanisms, eg, apomorphine; appetite-suppressant agents acting through histaminergic mechanisms (eg, histamine mimetics, H3 receptor modulators); enhancers of energy expenditure such as beta-3 adrenergic agonists and stimulators of uncoupling protein function; leptin and leptin mimetics; neuropeptide Y antagonists; melanocortin-1, 3 and 4 receptor modulators; cholecystokinin agonists; glucagon-like peptide-1 (GLP-1) mimetics and analogues (eg, Exendin); androgens (eg, dehydroepiandrosterone and derivatives such as etiocholandione), testosterone, anabolic steroids (eg, oxandrolone), and steroidal hormones; galanin receptor antagonists; cytokine agents such as ciliary neurotrophic factor; amylase inhibitors; enterostatin agonists/mimetics; orexin/hypocretin antagonists; urocortin antagonists; bombesin agonists; modulators of protein kinase A; corticotropin-releasing factor mimetics; cocaine- and amphetamine-regulated transcript mimetics; calcitonin-gene related peptide mimetics; and fatty acid synthase inhibitors.

There remains a need to provide an effective treatment for glucose-related disorders such as elevated glucose levels, Type 2 diabetes mellitus, Syndrome X, and the like. There also remains a need to provide an effective treatment for glucose related disorders which also slows or prevents the progression and / or development of Type 2 diabetes mellitus.

CN 104 447 722 discloses a canagliflozin anhydrous compound and a preparation method thereof. The disclosure also relates to application of the anhydrous crystal composition to preparation of drugs treating type 2 diabetes and related diseases.

CN 104 447 721 discloses a Canagliflozin anhydrous crystal form and a preparation method thereof. The disclosure also relates to application of the crystal form composition to preparation of medicaments for treating type 2 diabetes and related diseases.

CN 104 230 907 discloses crystal form III and crystal form IV of 1-(beta-D-glucopyranosyl)-4-methyl-3-[5-(4-fluorophenyl)-2-thienylmethyl, a preparation method of the crystal form III and the crystal form IV and a medicinal application of the crystal form III and the crystal form IV in treatment of diabetes mellitus.

CN 103936726 discloses crystal forms III and IV of 1-(b-D-glucopyranosyl)-4-methyl-3-[5-(4-fluorophenyl)-2-thienylmethyl]benzene. In an X-ray powder diffraction diagram, the disclosed crystal forms III and IV at least have characteristic peaks at 2q values being 6.61+/-0.2, 3.92+/-0.2 and 19.68+/-0.2, as well as 17.40+/-0.2, 15.35+/-0.2 and 14.91+/-0.2, respectively. Also disclosed is a preparation method and medical applications of the crystal. The disclosed crystal form III is an octanol complex.

## SUMMARY OF THE INVENTION

The present invention is directed to a process for the preparation of an anhydrous crystalline form of (1S)-1,5-anhydro-1-[3-[[5-(4-fluorophenyl)-2-thienyl]methyl]-4-methylphenyl]-D-glucitol comprising the following pXRD peaks (°2θ): 5.12 ± 0.02, 7.41 ± 0.02, 10.24 ± 0.02, 11.13 ± 0.02, 14.86 ± 0.02, and 30.01 ± 0.02, comprising dissolving a hemihydrate crystalline form of (1S)-1,5-anhydro-1-[3-[[5-(4-fluorophenyl)-2-thienyl]methyl]-4-methylphenyl]-D-glucitol in a dry organic solvent selected from the group consisting of isopropyl acetate and acetonitrile to yield a mixture; heating the mixture to a temperature in the range of from about 35°C to about solvent reflux temperature; and then cooling to about room temperature; to yield a precipitate of the anhydrous crystalline form.

The present invention is also directed to a process for the preparation of an anhydrous crystalline form of (1S)-1,5-anhydro-1-[3-[[5-(4-fluorophenyl)-2-thienyl]methyl]-4-methylphenyl]-D-glucitol comprising the following pXRD peaks (°2θ): 5.12 ± 0.02, 7.41 ± 0.02, 7.64 ± 0.02, 10.24 ± 0.02, 14.86 ± 0.02, 20.02 ± 0.02, 24.30 ± 0.02, 27.92 ± 0.02 and 30.01 ± 0.02, comprising dissolving a hemihydrate crystalline form of (1S)-1,5-anhydro-1-[3-[[5-(4-fluorophenyl)-2-thienyl]methyl]-4-methylphenyl]-D-glucitol in a dry organic solvent selected from the group consisting of isopropyl acetate and acetonitrile to yield a mixture; heating the mixture to a temperature in the range of from about 35°C to about solvent reflux temperature; and then cooling to about room temperature; to yield a precipitate of the anhydrous crystalline form.

The present invention is also directed to a process for the preparation of an anhydrous crystalline form of (1S)-1,5-anhydro-1-[3-[[5-(4-fluorophenyl)-2-thienyl]methyl]-4-methylphenyl]-D-glucitol comprising the following °2θ pXRD peaks:

| Position [°2θ ± 0.02] | d-Spacing [Å ± 0.02] |
|---|---|
| 14.86 | 5.96 |
| 15.29 | 5.79 |
| 17.32 | 5.12 |
| 18.27 | 4.86 |
| 18.47 | 4.80 |
| 20.48 | 4.34 |
| 27.92 | 3.20 |

comprising dissolving a hemihydrate crystalline form of (1S)-1,5-anhydro-1-[3-[[5-(4-fluorophenyl)-2-thienyl]methyl]-4-methylphenyl]-D-glucitol in a dry organic solvent selected from the group consisting of isopropyl acetate and acetonitrile to yield a mixture; heating the mixture to a temperature in the range of from about 35°C to about solvent reflux temperature; and then cooling to about room temperature; to yield a precipitate of the anhydrous crystalline form.

The present invention is also directed to a process for the preparation of an anhydrous crystalline form of (1S)-1,5-anhydro-1-[3-[[5-(4-fluorophenyl)-2-thienyl]methyl]-4-methylphenyl]-D-glucitol having substantially the same pXRD pattern as set forth in Figure 1, comprising dissolving a hemihydrate crystalline form of (1S)-1,5-anhydro-1-[3-[[5-(4-fluorophenyl)-2-thienyl]methyl]-4-methylphenyl]-D-glucitol in a dry organic solvent selected from the group consisting of isopropyl acetate and acetonitrile to yield a mixture; heating the mixture to a temperature in the range of from about 35°C to about solvent reflux temperature; and then cooling to about room temperature; to yield a precipitate of the anhydrous crystalline form.

The present invention is also directed to a process for the preparation of an anhydrous crystalline form of (1S)-1,5-anhydro-1-[3-[[5-(4-fluorophenyl)-2-thienyl]methyl]-4-methylphenyl]-D-glucitol, wherein the anhydrous crystalline form exhibits a peak melting point of about 126.77°C, comprising dissolving a hemihydrate crystalline form of (1S)-1,5-anhydro-1-[3-[[5-(4-fluorophenyl)-2-thienyl]methyl]-4-methylphenyl]-D-glucitol in a dry organic solvent selected from the group consisting of isopropyl acetate and acetonitrile to yield a mixture; heating the mixture to a temperature in the range of from about 35°C to about solvent reflux temperature; and then cooling to about room temperature; to yield a precipitate of the anhydrous crystalline form.

The present disclosure is directed to an anhydrous crystalline form of the compound of formula (I)

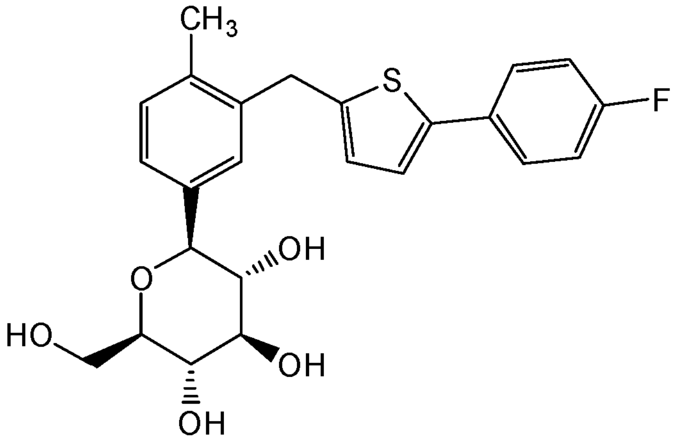

(I).

also known as (1S)-1,5-anhydro-1-[3-[[5-(4-fluorophenyl)-2-thienyl]methyl]-4-methylphenyl]-D-glucitol.

The present invention is further directed to processes for the preparation of the anhydrous crystalline form of the compound of formula (I), as herein described in more detail. Illustrative of the disclosure is a pharmaceutical composition comprising a pharmaceutically acceptable carrier and the anhydrous crystalline form of the compound of formula (I), as described herein. An illustration of the disclosure is a pharmaceutical composition made by mixing the anhydrous crystalline form of the compound of formula (I), as described herein and a pharmaceutically acceptable carrier. Illustrating the disclosure is a process for making a pharmaceutical composition comprising mixing the anhydrous crystalline form of the compound of formula (I), as described herein and a pharmaceutically acceptable carrier.

The present disclosure is further directed to methods for treating or delaying the progression or onset of diabetes mellitus (preferably Type 2 diabetes mellitus), diabetic complications (such as diabetic retinopathy, diabetic neuropathy, diabetic nephropathy), delayed wound healing, insulin resistance, hyperglycemia, hyperinsulinemia, elevated blood levels of fatty acid, elevate blood levels of glycerol, hyperlipidemia, obesity, hypertriglyceridemia, Syndrome X, atherosclerosis or hypertension, comprising administering to a subject in need thereof, a therapeutically effective amount of the anhydrous crystalline form of the compound of formula (I), as described herein

In certain embodiments, the present disclosure is directed to methods for the treatment and / or prevention of glucose-related disorders, said methods comprising administering to a subject in need thereof a therapeutically effective amount of the anhydrous crystalline form of the compound of formula (I) as described herein.

## BRIEF DESCRIPTION OF THE FIGURES

Figure 1 illustrates a representative pXRD pattern of the anhydrous crystalline form of (1S)-1,5-anhydro-1-[3-[[5-(4-fluorophenyl)-2-thienyl]methyl]-4-methylphenyl]-D-glucitol, measured as described herein.

Figure 2 illustrates a representative DSC for the anhydrous crystalline form of (1S)-1,5-anhydro-1-[3-[[5-(4-fluorophenyl)-2-thienyl]methyl]-4-methylphenyl]-D-glucitol, measured as described herein.

## DETAILED DESCRIPTION OF THE INVENTION

The present disclosure is directed to an anhydrous crystalline form of a compound of formula (I)

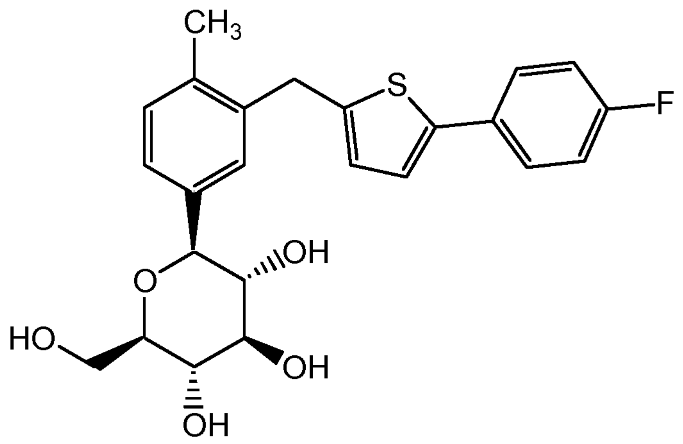

(I)

(also known as (1S)-1,5-anhydro-1-[3-[[5-(4-fluorophenyl)-2-thienyl]methyl]-4-methylphenyl]-D-glucitol). The compound of the formula (I) exhibits an inhibitory activity against sodium-dependent glucose transporter, such as for example SGLT2. The compound of formula (I) may be prepared,
for example, according to the process as disclosed in Nomura, S. et al., US Patent Publication, US 2005/0233988 A1, published October 20, 2005. A hemihydrate crystalline form of the compound of formula (I) may be prepared as disclosed in Nomura et al., US 2008/0146515 A1, published June 19, 2008, or Filliers, W., et al., US 2010/0099883 A1, published April 22, 2010.

The present disclosure is further directed to methods for the treatment and / or prevention of glucose-related disorders (preferably Type 2 diabetes mellitus), said methods comprising administering to a subject in need thereof the anhydrous crystalline form of the compound of formula (I), as described herein.

Where the compounds according to this invention have at least one **chiral center,** they may accordingly exist as enantiomers. Where the compounds possess two or more chiral centers, they may additionally exist as diastereomers. It is to be understood that all such isomers and mixtures thereof are encompassed within the scope of the present invention. Preferably, wherein the compound is present as an enantiomer, the enantiomer is present at an enantiomeric excess of greater than or equal to about 80%, more preferably, at an enantiomeric excess of greater than or equal to about 90%, more preferably still, at an enantiomeric excess of greater than or equal to about 95%, more preferably still, at an enantiomeric excess of greater than or equal to about 98%, most preferably, at an enantiomeric excess of greater than or equal to about 99%. Similarly, wherein the compound is present as a diastereomer, the diastereomer is present at an diastereomeric excess of greater than or equal to about 80%, more preferably, at an diastereomeric excess of greater than or equal to about 90%, more preferably still, at an diastereomeric excess of greater than or equal to about 95%, more preferably still, at an diastereomeric excess of greater than or equal to about 98%, most preferably, at an diastereomeric excess of greater than or equal to about 99%.

Furthermore, some of the crystalline forms for the compounds of the present disclosure may exist as polymorphs and as such are intended to be included in the present disclosure. In addition, some of the compounds of the present disclosure may form solvates with water (i.e., hydrates) or common organic solvents, and such solvates are also intended to be encompassed within the scope of this disclosure.

As used herein, unless otherwise noted, the term **"isolated form"** shall mean that the compound is present in a form which is separate from any solid mixture with another compound(s), solvent system or biological environment. In an embodiment of the present disclosure, the anhydrous crystalline form of the compound of formula (I) is present in an isolated form.

As used herein, unless otherwise noted, the term **"substantially pure form"** shall mean that the mole percent of impurities in the isolated crystalline form is less than about 5 mole percent, preferably less than about 2 mole percent, more preferably, less than about 0.5 mole percent, most preferably, less than about 0.1 mole percent. In an embodiment of the present disclosure, the anhydrous crystalline form of the compound of formula (I) is present as a substantially pure form.

The present disclosure is further directed to methods for the treatment and prevention of (preferably, the prevention of the development of) glucose related disorders comprising administering to a subject in need thereof a therapeutically effective amount of the anhydrous crystalline form of the compound of formula (I) as herein described.

The methods of the present disclosure are directed to the treatment and or prevention (including delay in the progression or onset) of "glucose-related disorders". As used herein, the term **"glucose related disorder"** shall be defined as any disorder which is characterized by or is developed as a consequence of elevated glucose levels. Glucose-related disorders shall include diabetes mellitus, diabetic complications (such as diabetic retinopathy, diabetic neuropathy, diabetic nephropathy), delayed wound healing, insulin resistance, hyperglycemia, hyperinsulinemia, elevated blood levels of fatty acids, elevated blood levels of glucose, hyperlipidemia, obesity, hypertriglyceridemia, Syndrome X, atherosclerosis, or hypertension. In particular, the "glucose related-disorder" is diabetes mellitus (type 1 and type 2 diabetes mellitus, etc.), diabetic complications (such as diabetic retinopathy, diabetic neuropathy, diabetic nephropathy), obesity, or postprandial hyperglycemia.

In an embodiment of the present disclosure, the glucose related disorder is selected from the group consisting of diabetes mellitus, diabetic complications (such as diabetic retinopathy, diabetic neuropathy, diabetic nephropathy), delayed wound healing, insulin resistance, hyperglycemia, hyperinsulinemia, elevated blood levels of fatty acids, hyperlipidemia, obesity, hypertriglyceridemia, Syndrome X, atherosclerosis and hypertension.

In another embodiment of the present disclosure, glucose related disorder is selected from the group consisting of type 1 diabetes mellitus, type 2 diabetes mellitus, diabetic retinopathy, diabetic neuropathy, diabetic nephropathy, obesity and postprandial hyperglycemia. In another embodiment of the present disclosure, the glucose related disorder is selected from the group consisting of type 1 diabetes mellitus, type 2 diabetes mellitus, diabetic retinopathy, diabetic neuropathy, diabetic nephropathy, obesity, and delayed wound healing. In another embodiment of the present disclosure, the glucose related disorders is selected from the group consisting of poor glycemic control, Type 2 Diabetes Mellitus, Syndrome X, gestational diabetes, insulin resistance, hyperglycemia. In another embodiment of the present disclosure, the glucose related disorder is Type 2 diabetes mellitus.

In another embodiment, the glucose related disorder is selected from the group consisting of elevated glucose level, pre-diabetes, impaired oral glucose tolerance, poor glycemic control, Type 2 Diabetes Mellitus, Syndrome X (also known as metabolic syndrome), gestational diabetes, insulin resistance, and hyperglycemia.

Treatment of glucose related disorders may comprise lowering glucose levels, improving glycemic control, decreasing insulin resistance and / or preventing the development of a glucose related disorder (for example preventing a patient suffering from impaired oral glucose tolerance or elevated glucose levels from developing Type 2 diabetes mellitus).

As used herein, the terms **"Syndrome X", "Metabolic Syndrome"** and **"Metabolic Syndrome X"** shall mean a disorder that presents risk factors for the development of Type 2 diabetes mellitus and cardiovascular disease and is characterized by insulin resistance and hyperinsulinemia and may be accompanied by one or more of the following: (a) glucose intolerance, (b) Type 2 diabetes mellitus, (c) dyslipidemia, (d) hypertension and (e) obesity.

As used herein, unless otherwise noted, the terms **"treating", "treatment"** and the like, shall include the management and care of a subject or patient (preferably mammal, more preferably human) for the purpose of combating a disease, condition, or disorder and includes the administration of a compound of the present disclosure to prevent the onset of the symptoms or complications, alleviate the symptoms or complications, or eliminate the disease, condition, or disorder.

As used herein, unless otherwise noted, the term **"prevention"** shall include (a) reduction in the frequency of one or more symptoms; (b) reduction in the severity of one or more symptoms; (c) the delay or avoidance of the development of additional symptoms; and / or (d) delay or avoidance of the development of the disorder or condition.

One skilled in the art will recognize that wherein the present disclosure is directed to methods of prevention, a subject in need of thereof (i.e. a subject in need of prevention) shall include any subject or patient (preferably a mammal, more preferably a human) who has experienced or exhibited at least one symptom of the disorder, disease or condition to be prevented. Further, a subject in need thereof may additionally be a subject (preferably a mammal, more preferably a human) who has not exhibited any symptoms of the disorder, disease or condition to be prevented, but who has been deemed by a physician, clinician or other medical profession to be at risk of developing said disorder, disease or condition. For example, the subject may be deemed at risk of developing a disorder, disease or condition (and therefore in need of prevention or preventive treatment) as a consequence of the subject’s medical history, including, but not limited to, family history, pre-disposition, co-existing (comorbid) disorders or conditions, genetic testing, and the like.

The term **"subject"** as used herein, refers to an animal, preferably a mammal, most preferably a human, who has been the object of treatment, observation or experiment. Preferably, the subject has experienced and / or exhibited at least one symptom of the disease or disorder to be treated and / or prevented.

The term **"therapeutically effective amount"** as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

Optimal dosages to be administered may be readily determined by those skilled in the art, and will vary with for example, the mode of administration, the strength of the preparation, the mode of administration, and the advancement of the disease condition. In addition, factors associated with the particular patient being treated, including patient age, weight, diet and time of administration, will result in the need to adjust dosages.

One skilled in the art will recognize that, both *in vivo* and *in vitro* trials using suitable, known and generally accepted cell and / or animal models are predictive of the ability of a test compound or co-therapy to treat or prevent a

given disorder. One skilled in the art will further recognize that human clinical trials including first-in-human, dose ranging and efficacy trials, in healthy patients and / or those suffering from a given disorder, may be completed according to methods well known in the clinical and medical arts.

As used herein, the term **"composition"** is intended to encompass a product comprising the specified ingredients in the specified amounts, as well as any product which results, directly or indirectly, from combinations of the specified ingredients in the specified amounts.

To provide a more concise description, some of the quantitative expressions herein are recited as a range from about amount X to about amount Y. It is understood that wherein a range is recited, the range is not limited to the recited upper and lower bounds, but rather includes the full range from about amount X through about amount Y, or any amount or range therein.

To provide a more concise description, some of the quantitative expressions given herein are not qualified with the term **"about".** It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

Examples of suitable solvents, bases, reaction temperatures, and other reaction parameters and components are provided in the detailed description which follows herein. One skilled in the art will recognize that the listing of said examples is not intended, and should not be construed, as limiting in any way the invention set forth in the claims which follow thereafter.

One skilled in the art will recognize that wherein a reaction step of the present invention may be carried out in a variety of solvents or solvent systems, said reaction step may also be carried out in a mixture of the suitable solvents or solvent systems.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

Additionally, chiral HPLC against a standard may be used to determine percent enantiomeric excess (%ee). The enantiomeric excess may be calculated as follows

$$[\ (\text{Rmoles}-\text{Smoles})/(\text{Rmoles}+\text{Smoles})\ ]\ \text{X}\ 100\%$$

where Rmoles and Smoles are the R and S mole fractions in the mixture such that Rmoles+Smoles = 1. The enantiomeric excess may alternatively be calculated from the specific rotations of the desired enantiomer and the prepared mixture as follows:

$$\text{ee} = ([\alpha\text{-obs}]\ /\ [\alpha\text{-max}])\ \text{X}\ 100.$$

Preparation of Anhydrous Crystalline Form:

The present disclosure is directed to an anhydrous crystalline form of the compound of formula (I). The anhydrous crystalline form of the compound of formula (I) may be prepared, for example, by recrystallization of the hemihydrate crystalline form of the compound of formula (I) from a suitably selected dry organic solvent selected from the group consisting of isopropyl acetate and acetonitrile, preferably isopropyl acetate; at a temperature in the range of from about 35°C to about solvent reflux temperature , or any temperature or range therein, preferably at a temperature in the range of from about 40°C to about 90°C, more preferably at a temperature in the range of from about 45°C to about 60°C, more preferably at a temperature of about 55°C.

Preferably, the hemihydrate crystalline form of the compound of formula (I), prepared for example as described in Filliers, W., et al., US 2010/0099883 A1, published April 22, 2010, is added to the suitably selected dry organic solvent, selected from the group consisting of isopropyl acetate and acetonitrile, preferably isopropyl acetate, and the resulting mixture is heated. (One skilled in the art will recognize that heating the mixture results in the compound of formula (I) dissolving in the dry organic solvent.) The heated mixture is then cooled to about room temperature, resulting in the

precipitation of the compound of formula (I) in the anhydrous crystalline form of the present invention.

In an embodiment of the present disclosure, the anhydrous crystalline form of the compound of formula (I) is present as needles.

pXRD and DSC Measurements:

The anhydrous crystalline form of the compound of formula (I) was characterized via powder X-ray diffraction (pXRD) and differential scanning calorimetry (DSC).

pXRD:

A solid sample of the anhydrous crystalline form of the present invention was analyzed using X-ray diffractometer, Philips Model Empyrean equipped with Empyrean goniometer, Empyrean Cu X-ray tube, and PIXcel detector with focusing parabolic X-ray mirror. The sample was scanned continuously from 3 to 40 °2θ at a step size of 0.0016413 °2θ and a time per step of 39.525 seconds (scan speed 0.010589°/sec). The x-ray tube voltage and current settings were 45 KV and 40 mA, respectively. The sample "as received" was packed on a zero background holder and scanned under ambient conditions of temperature and humidity. Incident beam and diffracted beam optics were as follows:

| **Incident Beam Optics:** | **Diffracted Beam Optics:** |
|---|---|
| PreFIX Module:<br>Programmable Divergence Slit Module | PreFIX Module:<br>X'Celerator Module |
| Offset = 0.000° | Offset = 0.000° |
| Filter: None | Filter: Nickel |
| Soller Slit: 0.04 Rad. | Soller Slit: 0.04 Rad. |
| Mask: Fixed 15 mm | |
| Anti-Scatter Slit: Fixed 1/2° | Anti-Scatter Slit: Programmable |

Anhydrous Crystalline Form pXRD:

The anhydrous crystalline form of the compound of formula (I) was characterized by powder X-ray diffraction (pXRD) according to the method as described above. Figure 1 which follows herein, illustrates a representative measured pXRD pattern for the anhydrous crystalline form of the compound of formula (I).

In an embodiment, the anhydrous crystalline form of the compound of formula (I) may be characterized by its powder X-ray diffraction pattern, comprising the peaks as listed in Table 1, below.

**Table 1: pXRD Peaks Characteristic of the Anhydrous Crystalline Form**

| **Position [°2θ ± 0.02]** | **d-Spacing [Å ± 0.02]** | **Relative Intensity [%]** |
|---|---|---|
| 5.12 | 17.27 | 3.28 |
| 7.41 | 11.94 | 5.67 |
| 7.64 | 11.58 | 2.86 |
| 8.05 | 10.99 | 2.88 |
| 10.24 | 8.64 | 16.77 |
| 11.13 | 7.95 | 2.14 |
| 12.09 | 7.32 | 0.58 |
| 12.80 | 6.91 | 1.44 |
| 13.16 | 6.73 | 5.31 |
| 14.86 | 5.96 | 26.65 |
| 15.29 | 5.79 | 23.08 |

(continued)

| Position [°2θ ± 0.02] | d-Spacing [Å ± 0.02] | Relative Intensity [%] |
|---|---|---|
| 16.11 | 5.50 | 9.12 |
| 16.64 | 5.33 | 0.76 |
| 17.32 | 5.12 | 100.00 |
| 18.27 | 4.86 | 59.07 |
| 18.47 | 4.80 | 35.97 |
| 18.76 | 4.73 | 5.79 |
| 19.05 | 4.66 | 6.92 |
| 20.02 | 4.43 | 6.54 |
| 20.48 | 4.34 | 23.71 |
| 21.24 | 4.18 | 18.74 |
| 21.87 | 4.06 | 0.59 |
| 22.37 | 3.97 | 17.61 |
| 22.90 | 3.88 | 9.05 |
| 23.38 | 3.80 | 3.85 |
| 23.62 | 3.77 | 2.64 |
| 24.30 | 3.66 | 6.26 |
| 24.61 | 3.62 | 0.62 |
| 25.25 | 3.53 | 0.83 |
| 25.75 | 3.46 | 2.02 |
| 26.46 | 3.37 | 1.46 |
| 27.08 | 3.29 | 2.92 |
| 27.92 | 3.20 | 36.27 |
| 28.49 | 3.13 | 5.37 |
| 28.75 | 3.11 | 1.25 |
| 29.16 | 3.06 | 2.42 |
| 29.51 | 3.03 | 2.37 |
| 30.01 | 2.98 | 6.25 |
| 30.51 | 2.93 | 2.42 |
| 31.07 | 2.88 | 1.44 |
| 31.66 | 2.83 | 6.64 |
| 32.28 | 2.77 | 1.24 |
| 33.37 | 2.68 | 4.81 |
| 33.72 | 2.66 | 1.42 |
| 34.49 | 2.60 | 3.43 |
| 35.34 | 2.54 | 0.62 |
| 35.71 | 2.51 | 1.09 |
| 36.35 | 2.47 | 2.08 |
| 37.05 | 2.43 | 2.06 |

(continued)

| Position [°2θ ± 0.02] | d-Spacing [Å ± 0.02] | Relative Intensity [%] |
|---|---|---|
| 38.17 | 2.36 | 2.45 |
| 39.15 | 2.30 | 2.39 |

In an embodiment, the anhydrous crystalline form of the compound of formula (I) is characterized by its pXRD pattern which comprises peaks having a relative intensity greater than or equal to about 5%, as shown in Table 2 below.

**Table 2: pXRD Peaks for the Anhydrous Crystalline Form**

| Position [°2θ ± 0.02] | d-Spacing [Å ± 0.02] | Relative Intensity [%] |
|---|---|---|
| 7.41 | 11.94 | 5.67 |
| 10.24 | 8.64 | 16.77 |
| 13.16 | 6.73 | 5.31 |
| 14.86 | 5.96 | 26.65 |
| 15.29 | 5.79 | 23.08 |
| 16.11 | 5.50 | 9.12 |
| 17.32 | 5.12 | 100.00 |
| 18.27 | 4.86 | 59.07 |
| 18.47 | 4.80 | 35.97 |
| 18.76 | 4.73 | 5.79 |
| 19.05 | 4.66 | 6.92 |
| 20.02 | 4.43 | 6.54 |
| 20.48 | 4.34 | 23.71 |
| 21.24 | 4.18 | 18.74 |
| 22.37 | 3.97 | 17.61 |
| 22.90 | 3.88 | 9.05 |
| 24.30 | 3.66 | 6.26 |
| 27.92 | 3.20 | 36.27 |
| 28.49 | 3.13 | 5.37 |
| 30.01 | 2.98 | 6.25 |
| 31.66 | 2.83 | 6.64 |

In another embodiment, the anhydrous crystalline form of the compound of formula (I) is characterized by its pXRD pattern which comprises peaks havinga relative intensity greater than or equal to about 10%, as shown in Table 3, below.

**Table 3: pXRD Peaks for the Anhydrous Crystalline Form**

| Position [°2θ ± 0.02] | d-Spacing [Å ± 0.02] | Relative Intensity [%] |
|---|---|---|
| 10.24 | 8.64 | 16.77 |
| 14.86 | 5.96 | 26.65 |
| 15.29 | 5.79 | 23.08 |

(continued)

| Position [°2θ ± 0.02] | d-Spacing [Å ± 0.02] | Relative Intensity [%] |
|---|---|---|
| 17.32 | 5.12 | 100.00 |
| 18.27 | 4.86 | 59.07 |
| 18.47 | 4.80 | 35.97 |
| 20.48 | 4.34 | 23.71 |
| 21.24 | 4.18 | 18.74 |
| 22.37 | 3.98 | 17.61 |
| 27.92 | 3.20 | 36.27 |

In another embodiment, the anhydrous crystalline form of the compound of formula (I) is characterized by its pXRD pattern which comprises peaks having a relative intensity greater than or equal to about 20 %, as shown in Table 4, below.

**Table 4: pXRD Peaks for the Anhydrous Crystalline Form**

| Position [°2θ ± 0.02] | d-Spacing [Å ± 0.02] | Relative Intensity [%] |
|---|---|---|
| 14.86 | 5.96 | 26.65 |
| 15.29 | 5.79 | 23.08 |
| 17.32 | 5.12 | 100.00 |
| 18.27 | 4.86 | 59.07 |
| 18.47 | 4.80 | 35.97 |
| 20.48 | 4.34 | 23.71 |
| 27.92 | 3.20 | 36.27 |

In another embodiment, the anhydrous crystalline form of the compound of formula (I) is characterized by the following pXRD peaks (°2θ ± 0.02): 5.12 ± 0.02, 7.41 ± 0.02, 7.64 ± 0.02, 10.24 ± 0.02, 11.13 ± 0.02, 12.80 ± 0.02, 14.86 ± 0.02, 20.02 ± 0.02, 24.30 ± 0.02, 27.08 ± 0.02, 27.92 ± 0.02 and 30.01 ± 0.02.

In another embodiment, the anhydrous crystalline form of the compound of formula (I) is characterized by the following pXRD peaks (°2θ ± 0.02): 5.12 ± 0.02, 7.41 ± 0.02, 7.64 ± 0.02, 10.24 ± 0.02, 14.86 ± 0.02, 20.02 ± 0.02, 24.30 ± 0.02, 27.92 ± 0.02 and 30.01 ± 0.02.

In another embodiment, the anhydrous crystalline form of the compound of formula (I) is characterized by the following pXRD peaks (°2θ ± 0.02): 5.12 ± 0.02, 7.41 ± 0.02, 10.24 ± 0.02, 11.13 ± 0.02, 14.86 ± 0.02, and 30.01 ± 0.02.

DSC:

Thermal analysis was performed using a TA instrument Model Q1000 DSC. The sample (~2-5 mg) was run in a covered without seal aluminum pan. The reference used was an empty aluminum pan. The sample was scanned from 25° to 250°C at a heating rate of 10°C/min with a nitrogen purge. The sample "as received" was placed in a TA Instruments aluminum Tzero sample pan and analyzed under nitrogen purge (50 ml/min).

The anhydrous crystalline form of the compound of formula (I) was further characterized using Differential Scanning Calorimetry (DSC), as shown in Figure 2, according to the method described above, and found to exhibit a single peak staring at 122.39°C (72.17 J/g) and with a peak at 126.77°C.

Pharmaceutical Composition and Methods of Treatment

The present disclosure further comprises pharmaceutical compositions comprising the anhydrous crystalline form of the compound of formula (I), as described herein, with a pharmaceutically acceptable carrier. Pharmaceutical

compositions containing the compound of the disclosure described herein as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare the pharmaceutical compositions of this disclosure, the compound of the present disclosure as the active ingredient is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, of from about 0.01 to about 1000 mg or any amount or range therein, and may be given at a dosage of from about 0.01 to about 500 mg/kg/day, or any amount or range therein, preferably from about 0.5 to about 100 mg/kg/day, or any amount or range therein. The dosages, however, may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present disclosure, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.01 to about 1,000 mg, or any amount or range therein, of the active ingredient of the present disclosure. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present disclosure may be incorporated for administration orally or by injection include, aqueous solutions, suitably flavoured syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinylpyrrolidone or gelatin.

The method of treating glucose-related disorders described in the present disclosure may also be carried out using a pharmaceutical composition comprising the compound as defined herein and a pharmaceutically acceptable carrier. The pharmaceutical composition may contain between about 0.01 mg and about 1000 mg of the compound, or any amount or range therein; preferably about 0.1 mg to about 500 mg of the compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixers, emulsions, and suspensions. Forms useful for parenteral administration include sterile solutions, emulsions and suspensions.

Advantageously, compounds of the present disclosure may be administered in a single daily dose, or the total daily dosage may be administered in divided doses of two, three or four times daily. Furthermore, compounds for the present disclosure can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methyl-cellulose and the like. For parenteral administration, sterile suspensions and solutions are desired. Isotonic preparations which generally contain suitable preservatives are employed when intravenous administration is desired.

To prepare a pharmaceutical composition of the present disclosure, the anhydrous crystalline form of the compound of formula (I) as the active ingredient is intimately admixed with a pharmaceutical carrier according to conventional pharmaceutical compounding techniques, which carrier may take a wide variety of forms depending of the form of preparation desired for administration (e.g. oral or parenteral). Suitable pharmaceutically acceptable carriers are well known in the art. Descriptions of some of these pharmaceutically acceptable carriers may be found in The Handbook of Pharmaceutical Excipients, published by the American Pharmaceutical Association and the Pharmaceutical Society of Great Britain.

Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

The anhydrous crystalline form of the compound of formula (I) of this disclosure may be administered in any of the foregoing compositions and according to dosage regimens established in the art whenever treatment of glucose-related is required.

The daily dosage of the products may be varied over a wide range from about 0.01 to about 1,000 mg per adult human per day, or any amount or range therein. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250 and 500 milligrams of the active ingredient for the symptomatic adjustment of the dosage to the patient to be treated.

Preferably, the anhydrous crystalline form of the compound of formula (I) is administered at a dosage level of from about 0.01 mg/kg to about 500 mg/kg of body weight per day, or 0.01 mg/kg to about 200 mg/kg of body weight per day, or any amount or range therein. Preferably, the range is from about 0.01 to about 50 mg/kg of body weight per day, or any amount or range therein, more preferably, from about 0.05 mg/kg to about 10 mg/kg, or any amount or range therein, more preferably, from about 1 to about 5 mg/kg of body weight per day, or any amount or range therein. In an embodiment, an effective amount of the anhydrous crystalline form of the compound of formula (I) is supplied at a dosage level of 10 mg, 25 mg, 50 mg, 100 mg, 150 mg or 300 mg, or any amount or range therein. The anhydrous crystalline form of the compound of formula (I) may be administered on a regimen of 1 to 4 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

One skilled in the art will recognize that, both *in vivo* and *in vitro* trials using suitable, known and generally accepted cell and / or animal models are predictive of the ability of a test compound to treat or prevent a given disorder.

One skilled in the art will further recognize that human clinical trails including first-in-human, dose ranging and efficacy trials, in healthy patients and / or those suffering from a given disorder, may be completed according to methods well known in the clinical and medical arts.

The following Examples are set forth to aid in the understanding of the invention, and are not intended and should not be construed to limit in any way the invention set forth in the claims which follow thereafter.

**Example 1**

**Anhydrous Crystalline Form of the Compound of Formula (I)**

Isopropyl acetate (4.96 vol/wt) was added to the compound of formula (I) prepared as described in Filliers, W., et al., US 2010/0099883 A1, published April 22, 2010 (1 wt/wt) and the resulting slurry was stirred 54°C overnight, then cooled to room temperature to yield a precipitate of the crystalline anhydride (as needles), which was isolated by filtration. (Yield: 80%)

**Example 2**

**Solid Oral Dosage Form - Prophetic Example**

As a specific embodiment of an oral composition, 100 mg of the anhydrous crystalline form of the compound of formula (I), prepared as described herein is formulated with sufficient finely divided lactose to provide a total amount of 580 to 590 mg to fill a size O hard gel capsule.

While the foregoing specification teaches the principles of the present invention, with examples provided for the purpose of illustration, it will be understood that the practice of the invention encompasses all of the usual variations, adaptations and/or modifications as come within the scope of the following claims and their equivalents.

**Claims**

1. A process for the preparation of an anhydrous crystalline form of (1S)-1,5-anhydro-1-[3-[[5-(4-fluorophenyl)-2-thienyl]methyl]-4-methylphenyl]-D-glucitol comprising the following pXRD peaks (°2θ): 5.12 ± 0.02, 7.41 ± 0.02, 10.24 ± 0.02, 11.13 ± 0.02, 14.86 ± 0.02, and 30.01 ± 0.02, comprising dissolving a hemihydrate crystalline form of (1S)-1,5-anhydro-1-[3-[[5-(4-fluorophenyl)-2-thienyl]methyl]-4-methylphenyl]-D-glucitol in a dry organic solvent selected from the group consisting of isopropyl acetate and acetonitrile to yield a mixture; heating the mixture to a temperature in the range of from about 35°C to about solvent reflux temperature; and then cooling to about room temperature; to yield a precipitate of the anhydrous crystalline form.

2. A process for the preparation of an anhydrous crystalline form of (1S)-1,5-anhydro-1-[3-[[5-(4-fluorophenyl)-2-thienyl]methyl]-4-methylphenyl]-D-glucitol comprising the following pXRD peaks (°2θ): 5.12 ± 0.02, 7.41 ± 0.02, 7.64 ± 0.02, 10.24 ± 0.02, 14.86 ± 0.02, 20.02 ± 0.02, 24.30 ± 0.02, 27.92 ± 0.02 and 30.01 ± 0.02, comprising dissolving a hemihydrate crystalline form of (1S)-1,5-anhydro-1-[3-[[5-(4-fluorophenyl)-2-thienyl]methyl]-4-methylphenyl]-D-glucitol in a dry organic solvent selected from the group consisting of isopropyl acetate and acetonitrile to yield a mixture; heating the mixture to a temperature in the range of from about 35°C to about solvent reflux temperature; and then cooling to about room temperature; to yield a precipitate of the anhydrous crystalline form.

3. A process of Claim 1 wherein the anhydrous crystalline form of (1S)-1,5-anhydro-1-[3-[[5-(4-fluorophenyl)-2-thienyl]methyl]-4-methylphenyl]-D-glucitol, comprises the following pXRD peaks (°2θ): 5.12 ± 0.02, 7.41 ± 0.02, 7.64 ± 0.02, 10.24 ± 0.02, 11.13 ± 0.02, 12.80 ± 0.02, 14.86 ± 0.02, 20.02 ± 0.02, 24.30 ± 0.02, 27.08 ± 0.02, 27.92 ± 0.02 and 30.01 ± 0.02.

4. A process for the preparation of an anhydrous crystalline form of (1S)-1,5-anhydro-1-[3-[[5-(4-fluorophenyl)-2-thienyl]methyl]-4-methylphenyl]-D-glucitol comprising the following °2θ pXRD peaks:

| Position [°2θ ± 0.02] | d-Spacing [Å ± 0.02] |
|---|---|
| 14.86 | 5.96 |
| 15.29 | 5.79 |

(continued)

| Position [°2θ ± 0.02] | d-Spacing [Å ± 0.02] |
|---|---|
| 17.32 | 5.12 |
| 18.27 | 4.86 |
| 18.47 | 4.80 |
| 20.48 | 4.34 |
| 27.92 | 3.20 |

comprising dissolving a hemihydrate crystalline form of (1S)-1,5-anhydro-1-[3-[[5-(4-fluorophenyl)-2-thienyl]methyl]-4-methylphenyl]-D-glucitol in a dry organic solvent selected from the group consisting of isopropyl acetate and acetonitrile to yield a mixture; heating the mixture to a temperature in the range of from about 35°C to about solvent reflux temperature; and then cooling to about room temperature; to yield a precipitate of the anhydrous crystalline form.

**5.** A process of Claim 3, wherein the anhydrous crystalline form comprises the following °2θ pXRD peaks:

| Position [°2θ ± 0.02] | d-Spacing [Å ± 0.02] |
|---|---|
| 10.24 | 8.64 |
| 14.86 | 5.96 |
| 15.29 | 5.79 |
| 17.32 | 5.12 |
| 18.27 | 4.86 |
| 18.47 | 4.80 |
| 20.48 | 4.34 |
| 21.24 | 4.18 |
| 22.37 | 3.98 |
| 27.92 | 3.20 |

**6.** A process of Claim 3, wherein the anhydrous crystalline form comprises the following °2θ pXRD peaks:

| Position [°2θ ± 0.02] | d-Spacing [Å ± 0.02] |
|---|---|
| 7.41 | 11.94 |
| 10.24 | 8.64 |
| 13.16 | 6.73 |
| 14.86 | 5.96 |
| 15.29 | 5.79 |
| 16.11 | 5.50 |
| 17.32 | 5.12 |
| 18.27 | 4.86 |
| 18.47 | 4.80 |
| 18.76 | 4.73 |
| 19.05 | 4.66 |

(continued)

| Position [°2θ ± 0.02] | d-Spacing [Å ± 0.02] |
|---|---|
| 20.02 | 4.43 |
| 20.48 | 4.34 |
| 21.24 | 4.18 |
| 22.37 | 3.97 |
| 22.90 | 3.88 |
| 24.30 | 3.66 |
| 27.92 | 3.20 |
| 28.49 | 3.13 |
| 30.01 | 2.98 |
| 31.66 | 2.83 |

**7.** A process of Claim 3, wherein the anhydrous crystalline form comprises the following °2θ pXRD peaks:

| Position [°2θ ± 0.02] | d-Spacing [Å ± 0.02] |
|---|---|
| 5.12 | 17.27 |
| 7.41 | 11.94 |
| 7.64 | 11.58 |
| 8.05 | 10.99 |
| 10.24 | 8.64 |
| 11.13 | 7.95 |
| 12.09 | 7.32 |
| 12.80 | 6.91 |
| 13.16 | 6.73 |
| 14.86 | 5.96 |
| 15.29 | 5.79 |
| 16.11 | 5.50 |
| 16.64 | 5.33 |
| 17.32 | 5.12 |
| 18.27 | 4.86 |
| 18.47 | 4.80 |
| 18.76 | 4.73 |
| 19.05 | 4.66 |
| 20.02 | 4.43 |
| 20.48 | 4.34 |
| 21.24 | 4.18 |
| 21.87 | 4.06 |
| 22.37 | 3.97 |
| 22.90 | 3.88 |

(continued)

| Position [°2θ ± 0.02] | d-Spacing [Å ± 0.02] |
|---|---|
| 23.38 | 3.80 |
| 23.62 | 3.77 |
| 24.30 | 3.66 |
| 24.61 | 3.62 |
| 25.25 | 3.53 |
| 25.75 | 3.46 |
| 26.46 | 3.37 |
| 27.08 | 3.29 |
| 27.92 | 3.20 |
| 28.49 | 3.13 |
| 28.75 | 3.11 |
| 29.16 | 3.06 |
| 29.51 | 3.03 |
| 30.01 | 2.98 |
| 30.51 | 2.93 |
| 31.07 | 2.88 |
| 31.66 | 2.83 |
| 32.28 | 2.77 |
| 33.37 | 2.68 |
| 33.72 | 2.66 |
| 34.49 | 2.60 |
| 35.34 | 2.54 |
| 35.71 | 2.51 |
| 36.35 | 2.47 |
| 37.05 | 2.43 |
| 38.17 | 2.36 |
| 39.15 | 2.30 |

**8.** A process for the preparation of an anhydrous crystalline form of (1S)-1,5-anhydro-1-[3-[[5-(4-fluorophenyl)-2-thienyl]methyl]-4-methylphenyl]-D-glucitol having substantially the same pXRD pattern as set forth in Figure 1, comprising dissolving a hemihydrate crystalline form of (1S)-1,5-anhydro-1-[3-[[5-(4-fluorophenyl)-2-thienyl]methyl]-4-methylphenyl]-D-glucitol in a dry organic solvent selected from the group consisting of isopropyl acetate and acetonitrile to yield a mixture; heating the mixture to a temperature in the range of from about 35°C to about solvent reflux temperature; and then cooling to about room temperature; to yield a precipitate of the anhydrous crystalline form.

**9.** A process for the preparation of an anhydrous crystalline form of (1S)-1,5-anhydro-1-[3-[[5-(4-fluorophenyl)-2-thienyl]methyl]-4-methylphenyl]-D-glucitol, wherein the anhydrous crystalline form exhibits a peak melting point of about 126.77°C, comprising dissolving a hemihydrate crystalline form of (1S)-1,5-anhydro-1-[3-[[5-(4-fluorophenyl)-2-thienyl]methyl]-4-methylphenyl]-D-glucitol in a dry organic solvent selected from the group consisting of isopropyl acetate and acetonitrile to yield a mixture; heating the mixture to a temperature in the range of from about 35°C to about solvent reflux temperature; and then cooling to about room temperature; to yield a precipitate of the anhydrous

crystalline form.

10. A process as in any of Claims 1-9, wherein the dry organic solvent is isopropyl acetate; and wherein the mixture is heated to a temperature in the range of from about 45°C to about 60°C.

**Patentansprüche**

1. Verfahren zur Herstellung einer wasserfreien kristallinen Form von (1S)-1,5-Anhydro-1-[3-[[5-(4-fluorphenyl)-2-thienyl]methyl]-4-methylphenyl]-D-glucitol mit den folgenden pXRD-Peaks (°2θ): 5,12 ± 0,02, 7,41 ± 0,02, 10,24 ± 0,02, 11,13 ± 0,02, 14,86 ± 0,02 und 30,01 ± 0,02, bei dem man eine kristalline Hemihydratform von (1S)-1,5-Anhydro-1-[3-[[5-(4-fluorphenyl)-2-thienyl]methyl]-4-methylphenyl]-D-glucitol in einem aus der aus Essigsäureisopropylester und Acetonitril bestehenden Gruppe ausgewählten trockenen organischen Lösungsmittel unter Erhalt einer Mischung löst, die Mischung auf eine Temperatur im Bereich von etwa 35°C bis etwa Lösungsmittelrückflusstemperatur erhitzt und dann auf etwa Raumtemperatur abkühlt, unter Erhalt eines Niederschlags der wasserfreien kristallinen Form.

2. Verfahren zur Herstellung einer wasserfreien kristallinen Form von (1S)-1,5-Anhydro-1-[3-[[5-(4-fluorphenyl)-2-thienyl]methyl]-4-methylphenyl]-D-glucitol mit den folgenden pXRD-Peaks (°2θ): 5,12 ± 0,02, 7,41 ± 0,02, 7,64 ± 0,02, 10,24 ± 0,02, 14,86 ± 0,02, 20,02 ± 0,02, 24,30 ± 0,02, 27,92 ± 0,02 und 30,01 ± 0,02, bei dem man eine kristalline Hemihydratform von (1S)-1,5-Anhydro-1-[3-[[5-(4-fluorphenyl)-2-thienyl]methyl]-4-methylphenyl]-D-glucitol in einem aus der aus Essigsäureisopropylester und Acetonitril bestehenden Gruppe ausgewählten trockenen organischen Lösungsmittel unter Erhalt einer Mischung löst, die Mischung auf eine Temperatur im Bereich von etwa 35°C bis etwa Lösungsmittelrückflusstemperatur erhitzt und dann auf etwa Raumtemperatur abkühlt, unter Erhalt eines Niederschlags der wasserfreien kristallinen Form.

3. Verfahren nach Anspruch 1, wobei die wasserfreie kristalline Form von (1S)-1,5-Anhydro-1-[3-[[5-(4-fluorphenyl)-2-thienyl]methyl]-4-methylphenyl]-D-glucitol die folgenden pXRD-Peaks (°2θ) umfasst: 5,12 ± 0,02, 7,41 ± 0,02, 7,64 ± 0,02, 10,24 ± 0,02, 11,13 ± 0,02, 12,80 ± 0,02, 14,86 ± 0,02, 20,02 ± 0,02, 24,30 ± 0,02, 27,08 ± 0,02, 27,92 ± 0,02 und 30,01 ± 0,02.

4. Verfahren zur Herstellung einer wasserfreien kristallinen Form von (1S)-1,5-Anhydro-1-[3-[[5-(4-fluorphenyl)-2-thienyl]methyl]-4-methylphenyl]-D-glucitol mit den folgenden °2θ-pXRD-Peaks:

| Position [°2θ ± 0,02] | d-Abstand [Å ± 0,02] |
|---|---|
| 14,86 | 5,96 |
| 15,29 | 5,79 |
| 17,32 | 5,12 |
| 18,27 | 4,86 |
| 18,47 | 4,80 |
| 20,48 | 4,34 |
| 27,92 | 3,20 |

bei dem man eine kristalline Hemihydratform von (1S)-1,5-Anhydro-1-[3-[[5-(4-fluorphenyl)-2-thienyl]methyl]-4-methylphenyl]-D-glucitol in einem aus der aus Essigsäureisopropylester und Acetonitril bestehenden Gruppe ausgewählten trockenen organischen Lösungsmittel unter Erhalt einer Mischung löst, die Mischung auf eine Temperatur im Bereich von etwa 35°C bis etwa Lösungsmittelrückflusstemperatur erhitzt und dann auf etwa Raumtemperatur abkühlt, unter Erhalt eines Niederschlags der wasserfreien kristallinen Form.

5. Verfahren nach Anspruch 3, wobei die wasserfreie kristalline Form die folgenden °2θ-pXRD-Peaks umfasst:

| Position [°2θ ± 0,02] | d-Abstand [Å ± 0,02] |
|---|---|
| 10,24 | 8,64 |
| 14,86 | 5,96 |
| 15,29 | 5,79 |
| 17,32 | 5,12 |
| 18,27 | 4,86 |
| 18,47 | 4,80 |
| 20,48 | 4,34 |
| 21,24 | 4,18 |
| 22,37 | 3,98 |
| 27,92 | 3,20 |

**6.** Verfahren nach Anspruch 3, wobei die wasserfreie kristalline Form die folgenden °2θ-pXRD-Peaks umfasst:

| Position [°2θ ± 0,02] | d-Abstand [Å ± 0,02] |
|---|---|
| 7,41 | 11,94 |
| 10,24 | 8,64 |
| 13,16 | 6,73 |
| 14,86 | 5,96 |
| 15,29 | 5,79 |
| 16,11 | 5,50 |
| 17,32 | 5,12 |
| 18,27 | 4,86 |
| 18,47 | 4,80 |
| 18,76 | 4,73 |
| 19,05 | 4, 66 |
| 20,02 | 4,43 |
| 20,48 | 4,34 |
| 21,24 | 4,18 |
| 22,37 | 3,97 |
| 22,90 | 3,88 |
| 24,30 | 3,66 |
| 27,92 | 3,20 |
| 28,49 | 3,13 |
| 30,01 | 2,98 |
| 31,66 | 2,83 |

**7.** Verfahren nach Anspruch 3, wobei die wasserfreie kristalline Form die folgenden °2θ-pXRD-Peaks umfasst:

| Position [°2θ ± 0,02] | d-Abstand [Å ± 0,02] |
|---|---|
| 5,12 | 17,27 |
| 7,41 | 11,94 |
| 7, 64 | 11,58 |
| 8,05 | 10,99 |
| 10,24 | 8,64 |
| 11,13 | 7,95 |
| 12,09 | 7,32 |
| 12,80 | 6,91 |
| 13,16 | 6,73 |
| 14,86 | 5,96 |
| 15,29 | 5,79 |
| 16,11 | 5,50 |
| 16,64 | 5,33 |
| 17,32 | 5,12 |
| 18,27 | 4,86 |
| 18,47 | 4,80 |
| 18,76 | 4,73 |
| 19,05 | 4, 66 |
| 20,02 | 4,43 |
| 20,48 | 4,34 |
| 21,24 | 4,18 |
| 21,87 | 4,06 |
| 22,37 | 3,97 |
| 22,90 | 3,88 |
| 23,38 | 3,80 |
| 23,62 | 3,77 |
| 24,30 | 3,66 |
| 24,61 | 3,62 |
| 25,25 | 3,53 |
| 25,75 | 3,46 |
| 26,46 | 3,37 |
| 27,08 | 3,29 |
| 27,92 | 3,20 |
| 28,49 | 3,13 |
| 28,75 | 3,11 |
| 29,16 | 3,06 |
| 29,51 | 3,03 |
| 30,01 | 2,98 |
| 30,51 | 2,93 |

(fortgesetzt)

| Position [°2θ ± 0,02] | d-Abstand [Å ± 0,02] |
|---|---|
| 31,07 | 2,88 |
| 31,66 | 2,83 |
| 32,28 | 2,77 |
| 33,37 | 2, 68 |
| 33,72 | 2,66 |
| 34,49 | 2,60 |
| 35,34 | 2,54 |
| 35,71 | 2,51 |
| 36,35 | 2,47 |
| 37,05 | 2,43 |
| 38,17 | 2,36 |
| 39,15 | 2,30 |

**8.** Verfahren zur Herstellung einer wasserfreien kristallinen Form von (1S)-1,5-Anhydro-1-[3-[[5-(4-fluorphenyl)-2-thienyl]methyl]-4-methylphenyl]-D-glucitol mit im Wesentlichen dem gleichen pXRD-Muster wie in Figur 1 gezeigt, bei dem man eine kristalline Hemihydratform von (1S)-1,5-Anhydro-1-[3-[[5-(4-fluorphenyl)-2-thienyl]methyl]-4-methylphenyl]-D-glucitol in einem aus der aus Essigsäureisopropylester und Acetonitril bestehenden Gruppe ausgewählten trockenen organischen Lösungsmittel unter Erhalt einer Mischung löst, die Mischung auf eine Temperatur im Bereich von etwa 35°C bis etwa Lösungsmittelrückflusstemperatur erhitzt und dann auf etwa Raumtemperatur abkühlt, unter Erhalt eines Niederschlags der wasserfreien kristallinen Form.

**9.** Verfahren zur Herstellung einer wasserfreien kristallinen Form von (1S)-1,5-Anhydro-1-[3-[[5-(4-fluorphenyl)-2-thienyl]methyl]-4-methylphenyl]-D-glucitol, wobei die wasserfreie kristalline Form einen Schmelzpunktpeak von etwa 126,77°C zeigt, bei dem man eine kristalline Hemihydratform von (1S)-1,5-Anhydro-1-[3-[[5-(4-fluorphenyl)-2-thienyl]methyl]-4-methylphenyl]-D-glucitol in einem aus der aus Essigsäureisopropylester und Acetonitril bestehenden Gruppe ausgewählten trockenen organischen Lösungsmittel unter Erhalt einer Mischung löst, die Mischung auf eine Temperatur im Bereich von etwa 35°C bis etwa Lösungsmittelrückflusstemperatur erhitzt und dann auf etwa Raumtemperatur abkühlt, unter Erhalt eines Niederschlags der wasserfreien kristallinen Form.

**10.** Verfahren nach einem der Ansprüche 1-9, wobei es sich bei dem trocknen organischen Lösungsmittel um Essigsäureisopropylester handelt und wobei die Mischung auf eine Temperatur im Bereich von etwa 45°C bis etwa 60°C erhitzt wird.

**Revendications**

**1.** Procédé pour la préparation d'une forme cristalline anhydre du (1S)-1,5-anhydro-1-[3-[[5-(4-fluorophényl)-2-thiényl]méthyl]-4-méthylphényl]-D-glucitol comprenant les pics de pXRD suivants (° à 2θ) : 5,12 ± 0,02, 7,41 ± 0,02, 10,24 ± 0,02, 11,13 ± 0,02, 14,86 ± 0,02, et 30,01 ± 0,02, comprenant la dissolution d'une forme cristalline d'hémihydrate du (1S)-1,5-anhydro-1-[3-[[5-(4-fluorophényl)-2-thiényl]méthyl]-4-méthylphényl]-D-glucitol dans un solvant organique sec choisi dans le groupe constitué par l'acétate d'isopropyle et l'acétonitrile pour donner un mélange ; le chauffage du mélange à une température dans la plage allant d'environ 35 °C à environ la température de reflux du solvant ; et ensuite le refroidissement approximativement à la température ambiante ; pour donner un précipité de la forme cristalline anhydre.

**2.** Procédé pour la préparation d'une forme cristalline anhydre du (1S)-1,5-anhydro-1-[3-[[5-(4-fluorophényl)-2-thiényl]méthyl]-4-méthylphényl]-D-glucitol comprenant les pics de pXRD suivants (° à 2θ) : 5,12 ± 0,02, 7,41 ± 0,02, 7,64 ± 0,02, 10,24 ± 0,02, 14,86 ± 0,02, 20,02 ± 0,02, 24,30 ± 0,02, 27,92 ± 0,02 et 30,01 ± 0,02, comprenant la dissolution d'une forme cristalline d'hémihydrate du (1S)-1,5-anhydro-1-[3-[[5-(4-fluorophényl)-2-thiényl]méthyl]-4-méthylphényl]-D-glucitol dans un solvant organique sec choisi dans le groupe constitué par l'acétate d'isopropyle et l'acétonitrile pour donner un mélange ; le chauffage du mélange à une température dans la plage allant d'environ 35 °C à environ la température de reflux du solvant ; et ensuite le refroidissement approximativement à la température ambiante ; pour donner un précipité de la forme cristalline anhydre.

**3.** Procédé selon la revendication 1, la forme cristalline anhydre du (1S)-1,5-anhydro-1-[3-[[5-(4-fluorophényl)-2-thiényl]méthyl]-4-méthylphényl]-D-glucitol comprenant les pics de pXRD suivants (° à 2θ) : 5,12 ± 0,02, 7,41 ± 0,02, 7,64 ± 0,02, 10,24 ± 0,02, 11,13 ± 0,02, 12,80 ± 0,02, 14,86 ± 0,02, 20,02 ± 0,02, 24,30 ± 0,02, 27,08 ± 0,02, 27,92 ± 0,02 et 30,01 ± 0,02.

**4.** Procédé pour la préparation d'une forme cristalline anhydre du (1S)-1,5-anhydro-1-[3-[[5-(4-fluorophényl)-2-thiényl]méthyl]-4-méthylphényl]-D-glucitol comprenant les pics de pXRD en ° à 2θ suivants :

| Position [° à 2θ ± 0,02] | Espacement d [Å ± 0,02] |
|---|---|
| 14,86 | 5,96 |
| 15,29 | 5,79 |
| 17,32 | 5,12 |
| 18,27 | 4,86 |
| 18,47 | 4,80 |
| 20,48 | 4,34 |
| 27,92 | 3,20 |

comprenant la dissolution d'une forme cristalline d'hémihydrate du (1S)-1,5-anhydro-1-[3-[[5-(4-fluorophényl)-2-thiényl]méthyl]-4-méthylphényl]-D-glucitol dans un solvant organique sec choisi dans le groupe constitué par l'acétate d'isopropyle et l'acétonitrile pour donner un mélange ; le chauffage du mélange à une température dans la plage allant d'environ 35 °C à environ la température de reflux du solvant ; et ensuite le refroidissement approximativement à la température ambiante ; pour donner un précipité de la forme cristalline anhydre.

**5.** Procédé selon la revendication 3, la forme cristalline anhydre comprenant les pics de pXRD en ° à 2θ suivants :

| Position [° à 2θ ± 0,02] | Espacement d [Å ± 0,02] |
|---|---|
| 10,24 | 8 64 |
| 14,86 | 5,96 |
| 15,29 | 5,79 |
| 17,32 | 5,12 |
| 18,27 | 4,86 |
| 18,47 | 4,80 |
| 20,48 | 4,34 |
| 21,24 | 4,18 |
| 22,37 | 3,98 |
| 27,92 | 3,20 |

**6.** Procédé selon la revendication 3, la forme cristalline anhydre comprenant les pics de pXRD en ° à 2θ suivants :

| Position [° à 2θ ± 0,02] | Espacement d [Å ± 0,02] |
|---|---|
| 7,41 | 11,94 |

(suite)

| Position [° à 2θ ± 0,02] | Espacement d [Å ± 0,02] |
|---|---|
| 10,24 | 8,64 |
| 13,16 | 6,73 |
| 14,86 | 5,96 |
| 15,29 | 5,79 |
| 16,11 | 5,50 |
| 17,32 | 5,12 |
| 18,27 | 4,86 |
| 18,47 | 4,80 |
| 18,76 | 4,73 |
| 19,05 | 4, 66 |
| 20,02 | 4,43 |
| 20,48 | 4,34 |
| 21,24 | 4,18 |
| 22,37 | 3,97 |
| 22,90 | 3,88 |
| 24,30 | 3,66 |
| 27,92 | 3,20 |
| 28,49 | 3,13 |
| 30,01 | 2,98 |
| 31,66 | 2,83 |

**7.** Procédé selon la revendication 3, la forme cristalline anhydre comprenant les pics de pXRD en ° à 2θ suivants :

| Position [° à 2θ ± 0,02] | Espacement d [Å ± 0,02] |
|---|---|
| 5,12 | 17,27 |
| 7,41 | 11,94 |
| 7, 64 | 11,58 |
| 8,05 | 10,99 |
| 10,24 | 8,64 |
| 11,13 | 7,95 |
| 12,09 | 7,32 |
| 12,80 | 6,91 |
| 13,16 | 6,73 |
| 14,86 | 5,96 |
| 15,29 | 5,79 |
| 16,11 | 5,50 |
| 16,64 | 5,33 |
| 17,32 | 5,12 |

(suite)

| Position [° à 2θ ± 0,02] | Espacement d [Å ± 0,02] |
|---|---|
| 18,27 | 4,86 |
| 18,47 | 4,80 |
| 18,76 | 4,73 |
| 19,05 | 4,66 |
| 20,02 | 4,43 |
| 20,48 | 4,34 |
| 21,24 | 4,18 |
| 21,87 | 4,06 |
| 22,37 | 3,97 |
| 22,90 | 3,88 |
| 23,38 | 3,80 |
| 23,62 | 3,77 |
| 24,30 | 3,66 |
| 24,61 | 3,62 |
| 25,25 | 3,53 |
| 25,75 | 3,46 |
| 26,46 | 3,37 |
| 27,08 | 3,29 |
| 27,92 | 3,20 |
| 28,49 | 3,13 |
| 28,75 | 3,11 |
| 29,16 | 3,06 |
| 29,51 | 3,03 |
| 30,01 | 2,98 |
| 30,51 | 2,93 |
| 31,07 | 2,88 |
| 31,66 | 2,83 |
| 32,28 | 2,77 |
| 33,37 | 2,68 |
| 33,72 | 2,66 |
| 34,49 | 2,60 |
| 35,34 | 2,54 |
| 35,71 | 2,51 |
| 36,35 | 2,47 |
| 37,05 | 2,43 |
| 38,17 | 2,36 |
| 39,15 | 2,30 |

8. Procédé pour la préparation d'une forme cristalline anhydre du (1S)-1,5-anhydro-1-[3-[[5-(4-fluorophényl)-2-thiényl]méthyl]-4-méthylphényl]-D-glucitol possédant sensiblement le même diagramme de pXRD tel qu'établi dans la figure 1, comprenant la dissolution d'une forme cristalline d'hémihydrate du (1S)-1,5-anhydro-1-[3-[[5-(4-fluorophényl)-2-thiényl]méthyl]-4-méthylphényl]-D-glucitol dans un solvant organique sec choisi dans le groupe constitué par l'acétate d'isopropyle et l'acétonitrile pour donner un mélange ; le chauffage du mélange à une température dans la plage allant d'environ 35 °C à environ la température de reflux du solvant ; et ensuite le refroidissement approximativement à la température ambiante ; pour donner un précipité de la forme cristalline anhydre.

9. Procédé pour la préparation d'une forme cristalline anhydre du (1S)-1,5-anhydro-1-[3-[[5-(4-fluorophényl)-2-thiényl]méthyl]-4-méthylphényl]-D-glucitol, la forme cristalline anhydre présentant un point de fusion maximal d'environ 126,77 °C, comprenant la dissolution d'une forme cristalline d'hémihydrate du (1S)-1,5-anhydro-1-[3-[[5-(4-fluorophényl)-2-thiényl]méthyl]-4-méthylphényl]-D-glucitol dans un solvant organique sec choisi dans le groupe constitué par l'acétate d'isopropyle et l'acétonitrile pour donner un mélange ; le chauffage du mélange à une température dans la plage allant d'environ 35 °C à environ la température de reflux du solvant ; et ensuite le refroidissement approximativement à la température ambiante ; pour donner un précipité de la forme cristalline anhydre.

10. Procédé selon l'une quelconque des revendications 1 à 9, le solvant organique sec étant l'acétate d'isopropyle ; et le mélange étant chauffé à une température dans la plage allant d'environ 45 °C à environ 60 °C.

FIG. 1

**Representative pXRD pattern of the anhydrous crystalline form of the compound of formula (I)**

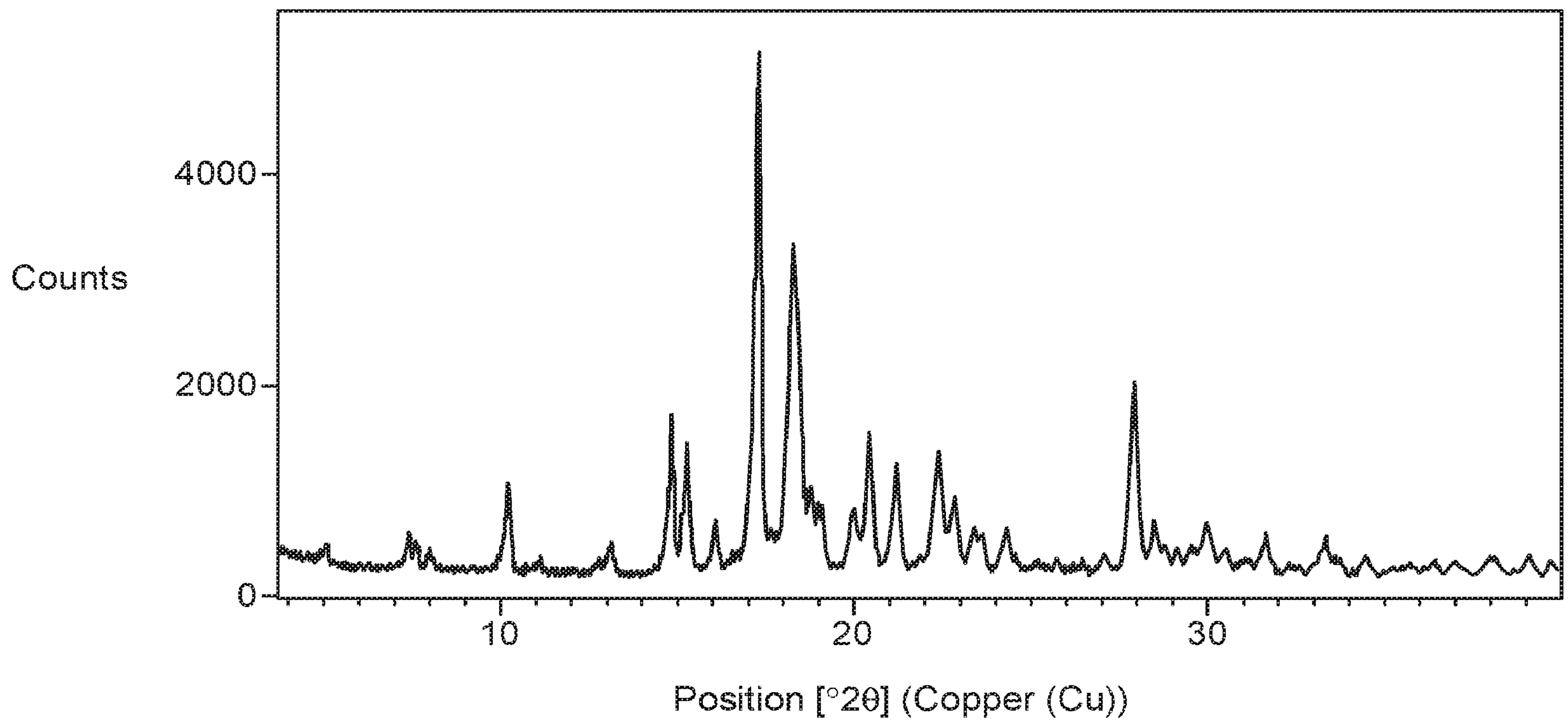

*FIG. 2*

**Representative DSC for the anhydrous crystalline form of the compound of formula (I)**

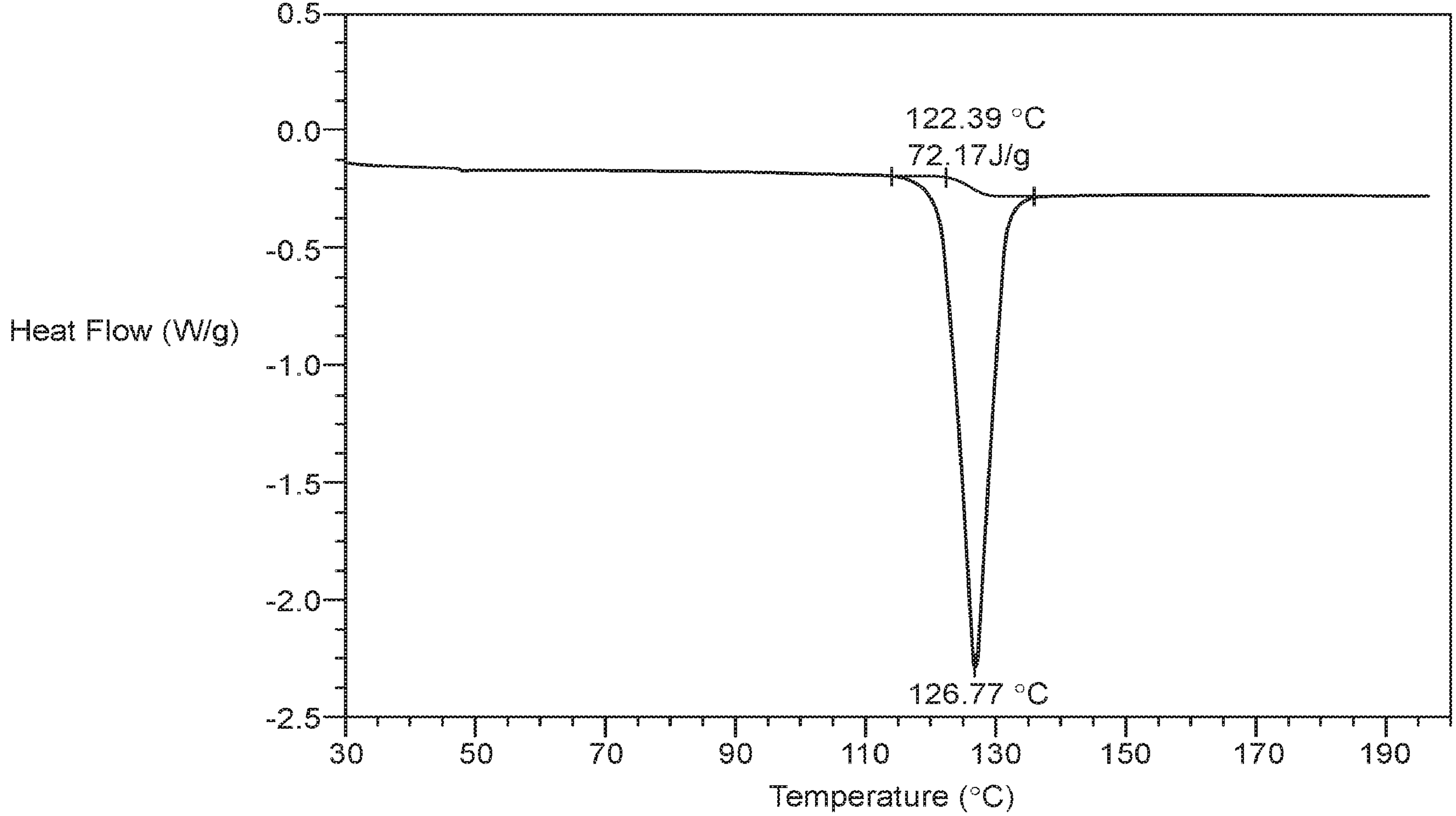

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62165265
- CN 104447722
- CN 104447721
- CN 104230907
- CN 103936726
- US 20050233988 A1
- US 20080146515 A1, Nomura
- US 20100099883 A1, Filliers, W.


### Non-patent literature cited in the description


- **EMANCIPATOR K.** *Am J Clin Pathol,* November 1999, vol. 112 (5), 665-674
- **CAUMO, A. et al.** *J Clin Endocrinol Metab,* 2000, vol. 85 (11), 4396-4402
- **HAFFNER, S.M.** *Diabet Med,* August 1997, vol. 14 (3), 12-8
- **GOLDBERG, R.B.** *Med Clin North Am,* July 1998, vol. 82 (4), 805-821
- **GROOP L et al.** *Am J Hypertens,* September 1997, vol. 10 (9), 172S-180S
- **HAFFNER, S.M.** *J Diabetes Complications,* March 1997, vol. 11 (2), 69-76
- **BECK-NIELSEN, H. et al.** *Diabet Med,* September 1996, vol. 13 (6), 78-84
- **DIUNNEEN, S.F.** 14. *Diabet Med,* August 1997, (3), 19-24
- **RAMLO-HALSTED, B.A. et al.** *Prim Care,* December 1999, vol. 26 (4), 771-789
- The Handbook of Pharmaceutical Excipients. American Pharmaceutical Association and the Pharmaceutical Society of Great Britain
- Pharmaceutical Dosage Forms: Tablets, Second Edition, Revised and Expanded. vol. 1-3
- Pharmaceutical Dosage Forms: Parenteral Medications. vol. 1-2
- Pharmaceutical Dosage Forms: Disperse Systems. Marcel Dekker, Inc, vol. 1-2